# EUROPÄISCHE PATENTSCHRIFT

(11) **EP 0 841 044 B1**
(45) Veröffentlichungstag und Bekanntmachung des Hinweises auf die Patenterteilung: **13.12.2000**
(21) Anmeldenummer: 97117948.6
(22) Anmeldetag: 16.10.1997
(51) Int. Cl.: A61F 5/01

(54) **Dynamische Gelenkstütze**
Dynamic brace
Dispositif de soutien dynamique

(30) Priorität: 31.10.1996 DE 19645076
(43) Veröffentlichungstag der Anmeldung: 13.05.1998
(73) Patentinhaber: Albrecht GmbH, 83071 Stephanskirchen (DE)
(72) Erfinder: Albrecht, Erich, D-83024 Rosenheim (DE); Opahle, Hans Georg, D-83024 Rosenheim (DE)
(74) Vertreter: Bauer, Friedrich, Dipl.-Ing.

(56) Entgegenhaltungen:
- WO-A-83/00283
- WO-A-95/23568
- US-A- 5 052 379
- US-A- 5 395 304
- US-A- 5 472 410

## Beschreibung

Die Erfindung betrifft eine Vorrichtung zur Reduktion von Streck- oder Beugedefiziten eines distalen Körpergliedes gegenüber einem angrenzenden, mit dem distalen Körperglied gelenkig verbundenen proximalen Körperglied, gemäß dem Oberbegriff des Patentanspruchs 1.

Insbesondere Gelenkskapseln und/oder Bindegewebe weisen beispielsweise nach Bänderoperationen, Unfällen, Entzündungen etc. häufig ein Streck- oder Beugedefizit auf. Dies bedeutet, daß ein distales Körperglied, beispielsweise ein Unterschenkel, nicht mehr vollständig in seine normale Extensions- oder Flexionslage bezüglich eines proximalen Körperglieds, beispielsweise eines Oberschenkels, gebracht werden kann.

Um einem derartigen Streck- oder Beugedefizit entgegenzuwirken, wird bekannterweise versucht, die Schrumpfungen wieder dadurch zu dehnen, daß das distale Körperglied bezüglich des proximalen Körperglieds mittels einer sogenannten Quengelvorrichtung in einer bestimmten Quengellage festgelegt oder in diese Quengellage mittels Federkraft vorgespannt wird.

Die Fixierung des Drehgelenkes in der Quengellage erfolgt bei bekannten Knieorthesen beispielsweise mittels Schrauben oder Stiften, welche in entsprechende Querbohrungen eingeführt werden können. Nachteilig ist bei diesen bekannten Knieorthesen jedoch, daß der Patient bei fixiertem Drehgelenk nicht oder nur mit großen Schwierigkeiten gehen kann, da er das Knie nicht abbiegen kann. Darüber hinaus ist das Entfernen bzw. Einsetzen der Arretierstife oder -schrauben mit einem gewissen Umstand verbunden. Von besonderer Bedeutung ist, daß die Fixierung von Ober- und Unterschenkel in der Quengellage häufig bereits nach kurzer Zeit Schmerzen verursacht, so daß die Quengellage nur kurze Zeit aufrecht erhalten werden kann. Der Erfolg dieser Methode bezüglich des Ausgleichs eines Streck- oder Beugedefizits läßt daher stark zu wünschen übrig.

Weiterhin ist bereits eine Quengelvorrichtung bekannt, bei der die distale Schiene permanent mittels Federvorspannung in die gewünschte Quengelrichtung vorgespannt ist. Nachteilig ist hierbei jedoch insbesondere, daß diese Quengelvorrichtung in der Regel jedesmal abgenommen werden muß, wenn das Gelenk in die zur Quengelrichtung entgegengesetzte Richtung bewegt werden soll, beispielsweise um die Bänder nach Auftreten von Schmerzen wieder zu entlasten, Untersuchungen vorzunehmen oder die betreffenden Körperglieder normal bewegen zu können. Wird die bekannte Quengelvorrichtung nicht abgenommen, sind die letztgenannten Aktivitäten meistens überhaupt nicht oder nur in stark eingeschränktem Umfang möglich.

Der Erfindung liegt die Aufgabe zugrunde, eine Vorrichtung gemäß dem Oberbegriff des Anspruches 1 zu schaffen, mit welcher einerseits auf möglichst einfache und wirkungsvolle Weise ein Streck- oder Beugedefizit eines distalen Körperglieds gegenüber einem proximalen Körperglied beseitig werden kann und die darüber hinaus dem Patienten mehr Bewegungsmöglichkeit und Tragekomfort bietet.

Diese Aufgabe wird erfindungsgemäß durch die Merkmale des Anspruches 1 gelöst. Vorteilhafte Ausführungsformen der Erfindung sind in den weiteren Ansprüchen beschrieben.

Bei der erfindungsgemäßen Vorrichtung ist ein Federkraftblockiermechanismus vorgesehen, der von einem nicht blokkierenden Zustand, in welchem die Vorspannkraft der Feder frei auf die distale Schiene übertragbar ist, in einen blockierenden Zustand bringbar ist, in welchem die Vorspannkraft der Feder von der distalen Schiene abgekoppelt ist und vom Federkraftblockiermechanismus aufgenommen wird, so daß die distale Schiene ohne Federeinwirkung über den freien Schwenkbereich schwenkbar ist.

Bei der erfindungsgemäßen Gelenkstütze übt die Feder eine kontinuierlich wirkende Vorspannkraft auf die distale Schiene aus, so daß diese permanent mit Federkraft in die gewünschte Quengelrichtung bis zu einem Endanschlag gezogen oder gedrückt wird, der die maximal zulässige Extension oder Flexion bestimmt. Die Position dieses Endanschlags kann vorzugsweise je nach dem gewünschten maximalen Schwenkbereich verändert werden. Besonders vorteilhaft ist es hierbei, wenn die Position des Endanschlags zumindest in einem bestimmten Winkelbereich stufenlos veränderbar ist, da dann die Schwenkbereichsbegrenzung und damit der Bereich des Quengelns sehr feinfühlig an den vorherrschenden Streck- oder Beugungsgrad des Patienten angepaßt und entsprechend nachgestellt werden kann (Controlled Passive Stretching - CPS). Die Federkraft bestimmt das Drehmoment, mit dem die distale Schiene gegenüber der proximalen Schiene gedreht wird. Trotz der Vorspannkraft der Feder besteht für den Patienten jedoch die Möglichkeit, das Gelenk im Bedarfsfall abzubiegen. Mit Hilfe des erfindungsgemäßen Federkraftblockiermechanismus ist es nämlich möglich, die Vorspannkraft der Feder vollkommen von der distalen Schiene abzukoppeln. Die distale Schiene kann in diesem Fall vollkommen frei von einer abgewinkelten Position bis zur maximalen Extensions- oder Flexionslage bewegt werden. Der Patient kann durch einfaches Umstellen des Federkraftblockiermechanismus zwischen einer Streck- oder Beugevorspannung und einer freien Beweglichkeit des Gelenkes wählen. Besonders vorteilhaft ist hierbei, daß auch bei abgekoppelter Federvorspannkraft die Einstellung der Vorspannkrafthöhe erhalten bleibt.

Vorteilhafterweise besteht die Feder aus einer die Schwenkachse umgebenden Spiralfeder, wobei ein Ende der Spiralfeder mit der einen Schiene und das andere Ende im freien Zustand der Spiralfeder mit der anderen Schiene in Wirkverbindung ist. Auf diese Weise läßt sich eine besonders platzsparende, kompakte Ausbildung bei gleichzeitig hoher Federkraft realisieren.

Zweckmäßigerweise ist eine mit einem Ende der Feder in Wirkverbindung stehende Vorspannkraftänderungseinrichtung vorgesehen, bei welcher die Relativposition dieses Federendes bezüglich des anderen Federendes veränderbar ist. Diese Vorspannkraftveränderungseinrichtung ist besonders vorteilhaft, da sie eine individuelle Anpassung der Federvorspannkraft an die Bedürfnisse des Patienten ermöglicht und das distale Körperglied mit einer optimierten Streck- oder Beugekraft in Extensions- bzw. Flexionsrichtung ziehen oder drücken kann.

Gemäß einer vorteilhaften Ausführungsform umfaßt die Vorspannkraftveränderungseinrichtung ein um die Schwenkachse drehbares und mittels eines Zahnradsperrmechanismus bezüglich einer Schiene fixierbares Zahnrad, an dem ein Ende der Feder in einem drehachsennahen Bereich befestigt ist. Die Vorspannkraft der Feder kann hierdurch auf einfache Weise durch Drehen und Arretieren des Zahnrades in einer neuen Position verändert werden.

Um das manuelle Drehen dieses Zahnrades, beispielsweise mittels eines Schraubendrehers, auch bei hohen Federkräften auf einfache Weise durchführen zu können, ist es vorteilhaft, wenn mit dem Zahnrad ein weiteres Betätigungszahnrad mit kleinerem Durchmesser zur Ausbildung eines Übersetzungsgetriebes in Eingriff steht.

Vorteilhafterweise umfaßt der Federkraftblockiermechanismus ein zwischen der Feder und der distalen Schiene angeordnetes Kopplungselement, das einerseits mit einem Ende der Feder verbunden und andererseits in Anlage an die distale Schiene bringbar ist. Das Kopplungselement drängt hierbei in seinem nicht blockierten Zustand die distale Schiene aufgrund der Vorspannkraft der Feder permanent in die gewünschte Drehrichtung, während es in seinem blokkierten Zustand daran gehindert ist, sich in diese Drehrichtung zu bewegen. Dies bedeutet, daß sich die distale Schiene bei blockiertem Kopplungselement vollkommen frei, d.h. losgelöst von der Federkraft, im verbleibenden Schwenkbereich bis zum Extensions- oder Flexionsendanschlag bewegen kann, falls der Patient beispielsweise das Gelenk normal bewegen möchte oder die Beweglichkeit des Körpergelenkes ohne Beeinflussung der Federkraft untersucht werden soll.

Diese Blockierung der Federkraft kann auf sehr einfache Weise dadurch bewirkt werden, wenn das Kopplungselement an seinem Außenumfang sich radial nach innen erstreckte Aussparungen aufweist, in die ein an der proximalen Schiene verschiebbar gehalterter Arretierungsstift einführbar ist. Dieser Arretierungsstift ist zweckmäßigerweise so ausgestaltet, daß er nach außen vorsteht und auf einfache Weise vom Patienten oder Arzt ergriffen und betätigt werden kann.

Die Erfindung wird nachfolgend anhand der Zeichnungen beispielhaft näher erläutert. In diesen zeigen:
- Figur 1 :: eine Draufsicht auf die erfindungsgemäße Vorrichtung;
- Figur 2 :: eine Unteransicht der Vorrichtung von Figur 1 in etwas vergrößertem Maßstab, wobei die freien Endbereiche der Schienen weggeschnitten sind;
- Figur 3 :: eine Unteransicht entsprechend Figur 2, wobei die proximale Schiene und ein Distanzstück entfernt sind;
- Figur 4 :: eine perspektivische Ansicht der erfindungsgemäßen Vorrichtung, wobei die freien Enden der proximalen und distalen Schiene weggeschnitten sind;
- Figur 5 :: eine Draufsicht auf den Mittelteil der Vorrichtung, wobei Teilbereiche freigeschnitten sind, um die Vorspannungkraftveränderungseinrichtung und den Zahnradsperrmechanismus zu zeigen;
- Figur 6 :: einen Vertikalschnitt längs der Linie VI-VI von Figur 5;
- Figur 7 :: eine Explosionsdarstellung der gesamten Vorrichtung ohne Befestigungsgurte; und
- Figur 8 :: eine perspektivisch gezeichnete Explosionsdarstellung der wichtigsten Teile zur Verdeutlichung der Übertragung bzw. Blokkierung der Federkraft zwischen der proximalen und distalen Schiene.

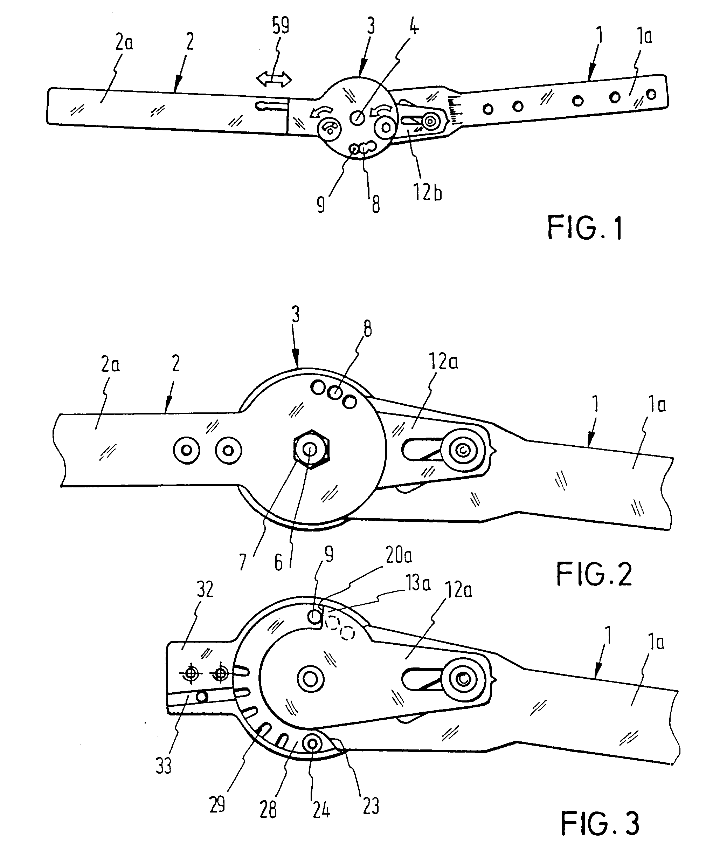

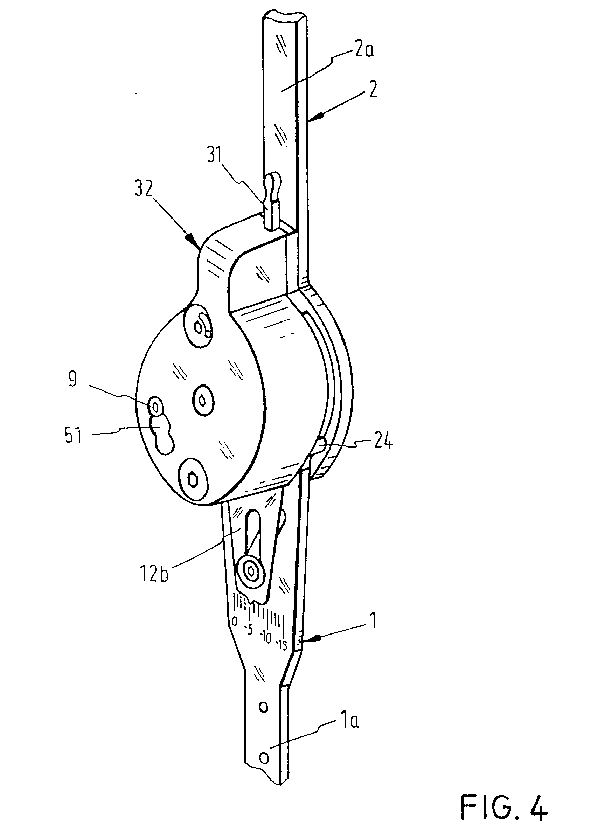

Im folgenden wird anhand der Figuren 1 bis 8 die erfindungsgemäße Vorrichtung beschrieben, wobei im Normalfall zwei Vorrichtungen verwendet werden, die sich auf gegenüberliegenden Seiten des Gelenkes befinden. Die auf der gegenüberliegenden Seite des Gelenkes anzuordnende Vorrichtung ist spiegelbildlich ausgebildet. Befestigungsgurte zur Befestigung der proximalen und distalen Schienen am proximalen bzw. distalen Körperglied sind der Übersichtlichkeit halber weggelassen. Die erfindungsgemäße Vorrichtung wird anhand des Beispiels einer Quengelvorrichtung für ein Knie beschrieben, könnte jedoch auch für andere Gelenke, beispielsweise für das Ellbogengelenk, Anwendung finden.

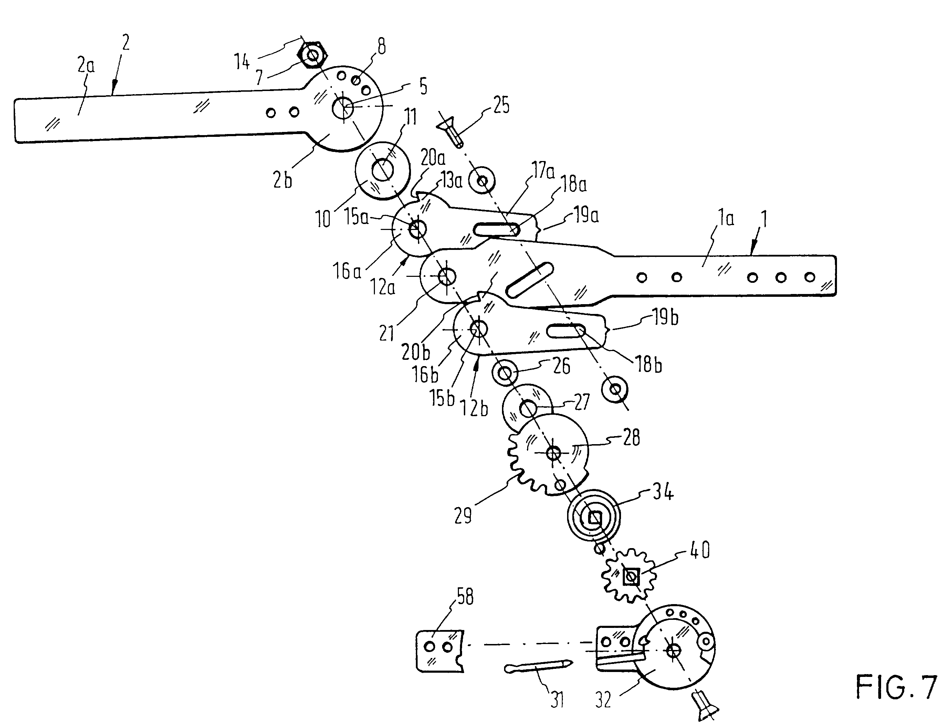

Wie aus Figur 1 und der Explosionsdarstellung von Figur 7 ersichtlich, weist die erfindungsgemäße Vorrichtung eine distale Schiene 1 mit einem Befestigungsabschnitt 1a auf, welcher mittels entsprechender, nicht gezeigter Gurte am Unterschenkel befestigt werden kann, sowie eine proximale Schiene 2 mit einem Befestigungsabschnitt 2a, welcher ebenfalls mittels nicht gezeigter Gurte am Oberschenkel befestigbar und über ein Drehgelenk 3 gelenkig mit der distalen Schiene 1 verbunden ist. Die Schwenkachse ist hierbei mit dem Bezugszeichen 14 bezeichnet.

Wie aus Figur 7 ersichtlich, weist ein kreisscheibenförmiger Gelenkabschnitt 2b der proximalen Schiene 2 in seinem Zentrum ein Loch 5 zum Durchtritt eines nur andeutungsweise gezeichneten Gelenkbolzens 6 auf, der auf der gegenüberliegenden Seite der proximalen Schiene 2 mittels einer Mutter 7 festgelegt werden kann. In der Nähe des Außenumfangs des Gelenkabschnittes 2b sind weiterhin drei Extensionsbegrenzungslöcher 8 vorgesehen, die in Umfangsrichtung nebeneinanderliegend angeordnet sind und zur Aufnahme eines in den Figuren 3, 4 und 8 gezeigten, als Schraube ausgebildeten Extensionsbegrenzungsstiftes 9 dienen. Zwei benachbarte Extensionsbegrenzungslöcher 8 sind in Umfangsrichtung jeweils mit einem Winkel von 15° versetzt angeordnet.

Die proximale Schiene 2 stellt das seitlich am weitesten innenliegende Teil der Vorrichtung dar, das somit, mit einer entsprechenden Polsterung und/oder an den Oberschenkel angepaßten Schalenteilen versehen, am Oberschenkel des Patienten anliegt.

Wie weiterhin aus Figur 7 ersichtlich, schließt an den Gelenkabschnitt 2b der proximalen Schiene 2 in Richtung nach außen eine kreisförmige Reibungsverminderungsscheibe 10 an, die beispielsweise aus einem dünnen Kunststoffblatt bestehen kann. Die Reibungsverminderungsscheibe 10 weist eine mittige Durchgangsöffnung 11 zum Durchtritt des Gelenkbolzens 6 auf.

In Richtung nach außen folgt eine Hälfte 12a eines Winkelverstellelementes 12, die an der Innenseite der distalen Schiene 1 und um die Schwenkachse 14 relativ beweglich an der Schiene 1 anliegt. Die andere Hälfte 12b ist identisch zur Hälfte 12a und liegt auf der gegenüberliegenden Seite der distalen Schiene 1 relativbeweglich an dieser an. Beide Hälften 12a, 12b weisen radial vorspringende Nasen 13a, 13b auf, die einen Brückenbereich bilden, indem sie miteinander verbunden sind.

Jede Winkelverstellelementhälfte 18a, 18b besteht aus einem dünnen, jedoch festen Stahlblechteil und ist schwenkbar um die Schwenkachse 14 herum gelagert, welche die gemeinsame Schwenkachse für alle Gelenkstützenteile bildet. Hierzu weisen die Hälften 12a, 12b eine Durchgangsöffnung 15a, 15b auf, durch welche der Gelenkbolzen 6 hindurchgeführt werden kann. Das proximale Ende der Hälften 12a, 12b ist in der Form eines halbkreisförmigen Abschnittes 16a, 16b ausgebildet, wobei der Radius dieses Halbkreises kleiner ist als der Radius des Kreisbogens, auf dem die Extensionsbegrenzungslöcher 8 der proximalen Schiene 2 liegen. Diese Löcher 8 werden somit vom halbkreisförmigen Abschnitt 16a, 16b der Winkelverstellelementhälften 18a, 18b nicht abgedeckt. Zweckmäßigerweise ist der Radius des halbkreisförmigen Abschnitts 16a, 16b gleich demjenigen der Reibungsverminderungsscheibe 10.

An den halbkreisförmigen Abschnitt 16a, 16b der Winkelverstellelementhälften 12a, 12b schließt sich ein länglicher Handhabungsabschnitt 17a, 17b an, in dem sich ein in Längsrichtung der Winkelverstellelementhälften 12a, 12b ausgerichtetes Langloch 18a, 18b befindet. Am distalen Ende des Handhabungsabschnittes 17a, 17b ist eine als Zeiger wirkende Spitze 19a, 19b vorgesehen.

Die bereits erwähnten Nasen 13a, 13b der Winkelverstellelementhälften 12a, 12b erstrecken sich in radialer Richtung über die Extensionsbegrenzungslöcher 8 hinaus nach außen, so daß eine stirnseitige Kontaktfläche 20a, 20b der Nasen 13a, 13b an dem Extensionsbegrenzungsstift 9 (Figur 3) anschlagen kann, welcher ein an der proximalen Schiene 2 befestigtes Anschlagelement zur Extensionsbegrenzung darstellt.

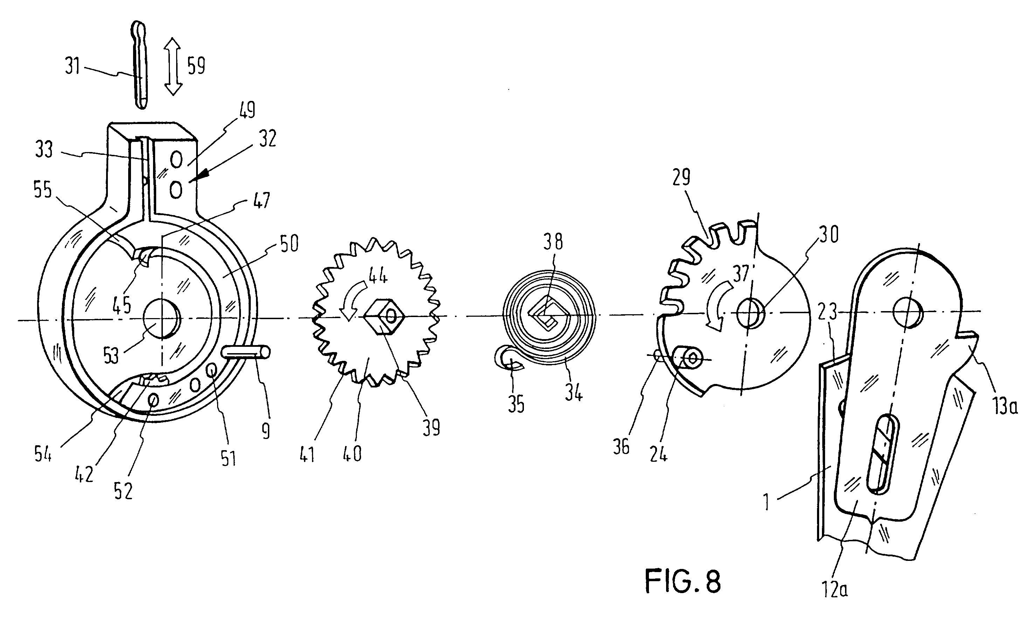

Wie weiterhin aus Figur 7 ersichtlich ist, weist die distale Schiene 1 eine Durchgangsöffnung 21 zum Hindurchführen des Gelenkbolzens 6 auf. Das benachbarte Ende ist wiederum etwa halbkreisförmig ausgebildet, wobei sich der Kreisbogen über etwas mehr als 180°, beispielsweise 220°, erstrecken kann. Der Radius dieses halbkreisförmigen Abschnittes entspricht wiederum demjenigen des halbkreisförmigen Abschnitts 16a, 16b der Winkelverstellelementhälften 12a, 12b. Nach dem im wesentlichen halbkreisförmigen Abschnitt verbreitert sich die distale Schiene 1, so daß eine stirnseitige Anschlagfläche 23 für einen später noch näher beschriebenen Mitnehmerbolzen 24 (Figuren 3 und 8) gebildet wird.

Wie aus Figur 7 ersichtlich, weist die distale Schiene 1 weiterhin ein schräg angeordnetes Langloch 22 auf, dessen Längsachse mit der Längsachse der distalen Schiene 1 im gezeigten Ausführungsbeispiel einen Winkel von 34° einschließt. Das Langloch 22 der distalen Schiene 1 verläuft somit auch schräg zu den Langlöchern 18a, 18b der Winkelverstellelementhälften 12a, 12b, wobei sich die Langlöcher 22; 18a, 18b überschneiden.

Aufgrund der gezeigten Anordnung der Langlöcher 22; 18a, 18b läßt sich die Winkelstellung der Winkelverstellelementhälften 12a, 12b relativ zur distalen Schiene 1 und damit die Extensionsbegrenzung stufenlos variieren, indem eine durch die Langlöcher 22; 18a, 18b hindurchgeführte Arretierschraube 25 längs der Langlöcher 22; 18a, 18b verschoben wird.

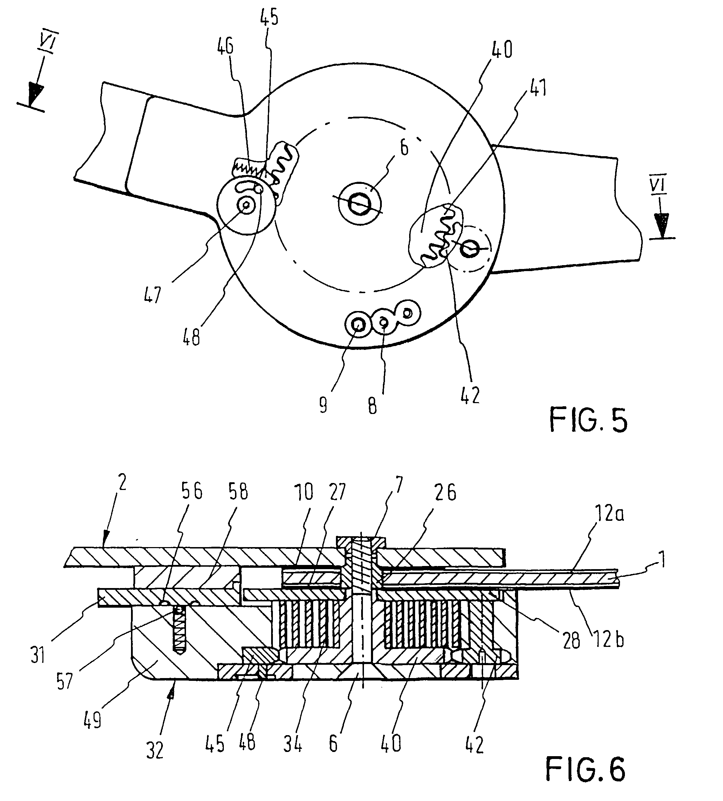

Wie weiterhin aus den Figuren 6 und 7 ersichtlich, ist eine relativ kleine Buchse 26 mit Innengewinde auf einen entsprechenden Gewindeabschnitt des Gelenkbolzens 6 aufschraubbar, um einerseits nachfolgend noch zu beschreibende Teile der Vorrichtung zusammenzuhalten und andererseits als Drehlager für die distale Schiene 1 und die Winkelverstellelementhälften 12a, 12b zu dienen.

An der Außenseite der äußeren Winkelverstellelementhälfte 12b schließt sich eine weitere Reibungsverminderungsscheibe 27 an, die in gleicher Weise wie die Reibungsverminderungsscheibe 10 ausgebildet ist.

An die Reibungsverminderungsscheibe 27 schließt sich ein scheibenförmiges Kopplungselement 28 an, das in Figur 8 näher gezeigt ist. Dieses Kopplungselement 28 weist über etwas mehr als die Hälfte seines Außenumfangs, beispielsweise 205°, einen Radius auf, der kleiner ist als der restliche Außenumfang, welcher eine Mehrzahl von nebeneinanderliegenden, sich radial nach innen erstreckenden Aussparungen 29 aufweist. In diesem letztgenannten Umfangsabschnitt ist das Kopplungselement 28 somit ähnlich wie ein Zahnrad mit Stirnverzahnung ausgebildet. Der bereits erwähnte Mitnehmerbolzen 24 (Figuren 3 und 8) ist ebenfalls in diesem Bereich mit größerem Durchmesser am Kopplungselement 28 befestigt und steht parallel zur Schwenkachse 14 über die Seitenfläche des Kopplungselements 28 vor. Der in Figur 7 dargestellte Gelenkbolzen 6 kann durch ein mittiges Durchgangsloch 30 des Kopplungselementes 28 hindurchgeführt werden.

In die Aussparung 29 kann zur Verhinderung einer Drehbewegung des Kopplungselements 28 ein Arretierstift 31 eingeschoben werden, welcher an einem proximalen Drehgelenkgehäuse 32 innerhalb einer Nut 33 längsverschiebbar gehaltert ist (Figur 8) .

Eine Feder 34 in der Form einer Spiralfeder schließt seitlich an das Kopplungselement 28 an. Das außenliegende freie Ende der Feder 34 ist als Kopplungsauge 35 ausgebildet, in das ein am Kopplungselement 28 befestigter Mitnehmerbolzen 36 einführbar ist. Dieser Mitnehmerbolzen 36 steht in entgegengesetzer Richtung wie der Mitnehmerbolzen 24 über das Kopplungselement 28 vor. Zweckmäßigerweise werden beide Mitnehmerbolzen 24, 36 durch eine einzige Inbusschraube gebildet, die durch eine entsprechende Gewindebohrung des Kopplungselementes 28 so hineingeschraubt wird, daß der Gewindeschaft auf der anderen Seite des Kopplungselementes 28 übersteht.

Im zusammengebauten Zustand übt die Feder 34 über den Mitnehmerbolzen 36 eine permanente Vorspannkraft auf das Kopplungselement 28 aus, welche dieses in Richtung des Pfeiles 37 zu drehen versucht.

Das innenliegende freie Ende der Feder 34 ist in der Form eines mittigen viereckigen Durchgangslochs 38 gewickelt, so daß die Feder 34 auf einen entsprechenden viereckigen mittigen Haltezapfen 39 eines Zahnrades 40 drehfest aufgeschoben werden kann. Das Zahnrad 40 weist eine Stirnverzahnung 41 auf, welche mit einer entsprechenden Verzahnung eines wesentlich kleineren Betätigungszahnrades 42 in Eingriff ist (Figuren 5 und 6). Dieses Betätigungszahnrad 42 ist in einem außenumfangsnahen Bereich des Drehgelenkgehäuses 32 drehbar gelagert und kann über eine entsprechende Öffnung 43 im Drehgelenkgehäuse 32 von außen her, beispielsweise mittels eines Inbusschlüssels, verdreht werden.

Das Betätigungszahnrad 42 bildet zusammen mit dem Zahnrad 40 ein Übersetzungsgetriebe, um mittels des Haltezapfens 39 die Feder 34 über ihr inneres Ende in Richtung des Pfeiles 44 stärker aufzuwickeln und dadurch die Vorspannkraft zu erhöhen.

Um zu gewährleisten, daß das Zahnrad 40 nach Einstellen der gewünschten Vorspannkraft in der gewünschten Position bleibt und sich nicht entgegen der Richtung des Pfeiles 44 zurückdreht, ist im Drehgelenkgehäuse 32 ein Zahnradsperrmechanismus in der Form einer Sperrklinke 45 schwenkbar gelagert. Die Sperrklinke 45 ist als Schwenkhebel ausgebildet und greift aufgrund der Vorspannkraft einer Sperrklinkenfeder 46 (Figur 5) in die Stirnverzahnung 41 des Zahnrades 40 ein. Die Schwenkachse für die Sperrklinke ist in Figur 5 mit 47 bezeichnet. An der Sperrklinke 45 ist weiterhin ein Betätigungsstift 48 vorgesehen, der von außen her durch eine entsprechende Öffnung im Drehgelenkgehäuse 32 hindurch betätigbar ist, um die Sperrklinke 45 entgegen der Kraft der Sperrklinkenfeder 46 außer Eingriff mit dem Zahnrad 40 zu bringen.

Wie aus Figur 8 ersichtlich, ist das Drehgelenkgehäuse 32, welches fest mit der in Figur 8 nicht dargestellten proximalen Schiene 2 verbunden ist, im wesentlichen in der Form einer kreisrunden Schüssel ausgebildet, welche einen seitlich vorstehenden, massiven Befestigungsansatz 49 zur Befestigung der proximalen Schiene 2 aufweist. Von der seitlichen Innenwand der kreisrunden Schüssel erstreckt sich ein massiver Steg 50 radial nach innen, der sich vom Bereich des Befestigungsansatzes 49 in Umfangsrichtung über einen Winkel von etwa 250° erstreckt. Im Bereich dieses Steges 50 sind drei in Umfangsrichtung nebeneinanderliegende, durchgehende Extensionsbegrenzungslöcher 51 vorgesehen, die den Extensionsbegrenzungslöchern 8 der proximalen Schiene 2 (Figuren 2 und 7) genau gegenüberliegen und zum Einschrauben des Extensionsbegrenzungsstiftes 9 dienen. Die Schwenkachse 52 für das Betätigungszahnrad 42 und die Schwenkachse 47 für die Sperrklinke 45 sind ebenfalls im Bereich des Steges 50 gelagert.

Der vertiefte Bereich radial innerhalb des Steges 50, d.h. der Bereich um ein mittiges Durchgangsloch 53 herum, dient zur Aufnahme des Zahnrades 40 und der darauf aufgesteckten Feder 34, wie auch aus dem Vertikalschnitt von Figur 6 ersichtlich ist. Das vorstehende freie äußere Ende der Feder 34 mit dem Auge 35 und dem darin aufgenommenen Mitnehmerbolzen 36 des Kopplungselementes 28 liegen hierbei in demjenigen seitennahen Bereich innerhalb des Drehgelenkgehäuses 32, über den sich der Steg 50 nicht erstreckt, d.h. im Bereich zwischen den Stirnwänden 54 und 55 des Steges 50. In der maximal zulässigen Extensionsstellung der distalen Schiene 1, die in Figur 8 gezeigt ist, befindet sich das Auge 35 der Feder 34 in der Nähe der Stirnwand 54. Wird das Knie abgewinkelt, d.h. in die Flexionsstellung bewegt, bewegt sich das Auge 35 der Feder 34 in Richtung der gegenüberliegenden Stirnwand 55 des Steges 50.

Aus Figur 6 ist weiterhin ersichtlich, daß der in der Nut 33 liegende Arretierstift 31 an seiner Unterseite 2 beabstandete Einkerbungen 56 aufweist, um den Arretierstift 31 in seinen Endstellungen durch Eindrücken einer federbeaufschlagten Rastkugel 57 zu haltern.

Wie weiterhin aus Figur 6 ersichtlich, ist zwischen dem Befestigungsansatz 49 des Drehgelenkgehäuses 32 und der proximalen Schiene 2 ein Distanzstück 58 vorgesehen, um zwischen der proximalen Schiene 2 und dem Kopplungselement 28 einen genügenden Zwischenraum zur Aufnahme der distalen Schiene 1 einzuhalten. Die proximale Schiene 2 wird durch das Distanzstück 58 hindurch mit dem Befestigungsansatz 49 fest verschraubt.

Die Funktionsweise der dargestellten Gelenkstütze wird im folgenden unter Bezugnahme auf Figur 8 erläutert.

Im Normalzustand drängt die Feder 34, welche mit ihrem inneren Ende über den Haltezapfen 39 des Zahnrades 40 innerhalb des Drehgelenkgehäuses 32 festgelegt ist, das Kopplungselement 28 über den Mitnehmerbolzen 36 zu einer Drehbewegung in Richtung des Pfeiles 37, wobei in Figur 8 bereits die maximal mögliche Schwenklage in dieser Richtung gezeigt ist. Voraussetzung hierfür ist allerdings, daß die Spitze des Arretierstiftes 31 nicht in den schüsselförmigen Teil des Drehgelenkgehäuses 32 und damit in eine der Aussparungen 29 des Kopplungselementes 28 hineinragt, sondern soweit zurückgezogen ist, daß die Schwenkbewegung des Kopplungselementes 28 nicht behindert wird. In diesem Zustand liegt der Mitnehmerbolzen 24 des Kopplungsteiles 28 permanent an der Anschlagfläche 23 der distalen Schiene 1 an, so daß auf diese permanent ein Drehmoment im Gegenuhrzeigersinn, d.h. in Extensionsrichtung, ausgeübt wird. Diese Schwenkbewegung dauert an, bis die Nasen 13a, 13b des Winkelverstellelementes 12 am Extensionsbegrenzungsstift 9 anschlagen, der sich quer durch das gesamte Drehgelenk erstreckt. Gleichzeitig ist jedoch ein Verschwenken der distalen Schiene 1 gegenüber dem Drehgelenkgehäuse 32 und damit gegenüber der proximalen Schiene 2 im Uhrzeigersinn, d.h. in Flexionsrichtung, möglich, wobei die Vorspannkraft der Feder 34 überwunden werden muß.

Soll die Vorspannkraft der Feder 34 auf die distale Schiene 1 aufgehoben werden, wird die distale Schiene 1 zunächst soweit in Flexionsrichtung, d.h. in Figur 8 im Uhrzeigersinn, geschwenkt, bis eine der Aussparungen 29 des Kopplungselementes 28 zum Arretierstift 31 ausgerichtet ist und dieser in die entsprechende Aussparung 29 eingeschoben werden kann. Dieses Verschieben des Arretierstiftes 31 wird in den Figuren 1 und 8 mit einem Doppelpfeil 59 veranschaulicht. Ist die weitere Schwenkbewegung des Kopplungselementes 28 auf diese Weise blockiert, kann sich die distale Schiene 1 vom Mitnehmerbolzen 24 lösen und frei bis zur maximalen Extensionslage bewegen. Es ist erkennbar, daß der vorspannkraftfreie Schwenkbereich der distalen Schiene um so größer ist, je weiter diese vor dem Einrücken des Arretierstiftes 31 in Flexionsrichtung zurückgeschwenkt worden ist. Wird der Arretierstift 31 in diejenige Aussparung 29 des Kopplungselementes 28 eingerückt, die den Mitnehmerbolzen 24, 36 am nächsten liegt, beträgt der federkraftfreie Schwenkbereich der distalen Schiene 1 etwa 90°.

Um von einer federkraftfreien wieder zu einer federbelasteten Verschwenkung zu kommen, muß lediglich der Arretierstift 31 aus der Aussparung 29 zurückgezogen und dadurch die freie Verschwenkung des Kopplungselementes 28 in Richtung des Pfeiles 37 ermöglicht werden.

Die erfindungsgemäße Vorrichtung wurde anhand einer Quengelvorrichtung beschrieben, bei welcher die Vorspannkraft der Feder 34 in Extensionsrichtung wirkt und Extensionsendanschläge 9; 13a, 13b vorgesehen sind. Alternativ kann die erfindungsgemäße Vorrichtung auch ohne weiteres derart ausgebildet werden, daß die Feder 34 in Flexionsrichtung wirkt und entsprechende Flexionsendanschläge vorhanden sind.

## Patentansprüche

1. Vorrichtung zur Reduktion von Streck- oder Beugedefiziten eines distalen Körperglieds gegenüber einem angrenzenden, mit dem distalen Körperglied gelenkig verbundenen proximalen Körperglied, mit am distalen und proximalen Körperglied befestigbaren Schienen (1, 2), die über ein Drehgelenk (3) miteinander verbunden sind, wobei im Bereich des Drehgelenks (3) eine zwischen der distalen und proximalen Schiene (1, 2) wirkende Feder (34) vorgesehen ist, welche im freien Zustand der Feder (34) auf die distale Schiene (1) eine Vorspannkraft in einer vorbestimmten Drehrichtung ausübt, **dadurch gekennzeichnet**, daß ein Federkraftblockiermechanismus (28, 29, 31) vorgesehen ist, der von einem nichtblockierenden Zustand, in welchem die Vorspannkraft der Feder (34) frei auf die distale Schiene (1) übertragbar ist, in einen blockierenden Zustand bringbar ist, in welchem die Vorspannkraft der Feder (34) von der distalen Schiene (1) abgekoppelt ist und vom Federkraftblockiermechanismus (28, 29, 31) aufgenommen wird, so daß die distale Schiene (1) ohne Federeinwirkung über den freien Schwenkbereich schwenkbar ist.

2. Vorrichtung nach Anspruch 1, **dadurch gekennzeichnet**, daß die Feder (34) aus einer die Schwenkachse (14) umgebenden Spiralfeder besteht, wobei ein Ende der Spiralfeder mit der einen Schiene und das andere Ende im freien Zustand der Spiralfeder mit der anderen Schiene in Wirkverbindung ist.

3. Vorrichtung nach Anspruch 1 oder 2, **dadurch gekennzeichnet**, daß eine mit einem Ende der Feder (34) in Wirkverbindung stehende Vorspannkraftveränderungseinrichtung (40, 42) vorgesehen ist, mit welcher die Relativposition dieses Federendes bezüglich des anderen Federendes veränderbar ist.

4. Vorrichtung nach Anspruch 3, **dadurch gekennzeichnet**, daß die Vorspannkraftveränderungseinrichtung (40, 42) ein um die Schwenkachse (14) drehbares und mittels eines Zahnradsperrmechanismus (45) bezüglich einer Schiene (2) fixierbares Zahnrad (40) umfaßt, an dem ein Ende der Feder (34) in einem drehachsennahen Bereich befestigt ist.

5. Vorrichtung nach Anspruch 4, **dadurch gekennzeichnet**, daß der Zahnradsperrmechanismus aus einer in die Verzahnung (41) des Zahnrads (40) eingreifenden Sperrklinke (45) besteht, welche die Umdrehung des Zahnrads (40) in einer Drehrichtung blockiert und in der anderen Drehrichtung zuläßt.

6. Vorrichtung nach Anspruch 4 oder 5, **dadurch gekennzeichnet**, daß mit dem Zahnrad (40) ein weiteres Betätigungszahnrad (42) mit kleinerem Durchmesser zur Ausbildung eines Übersetzungsgetriebes in Eingriff steht.

7. Vorrichtung nach einem der vorhergehenden Ansprüche, **dadurch gekennzeichnet**, daß der Federkraftblockiermechanismus ein zwischen der Feder (34) und der distalen Schiene (1) angeordnetes Kopplungselement (28) umfaßt, das einerseits mit einem Ende der Feder (34) verbunden und andererseits in Anlage an die distale Schiene (1) bringbar ist, wobei das Kopplungselement in seinem nichtblockierten Zustand die distale Schiene (1) aufgrund der Vorspannkraft der Feder (34) permanent in die gewünschte Drehrichtung drängt, während es in seinem blockierten Zustand daran gehindert ist, sich in diese Drehrichtung zu bewegen.

8. Vorrichtung nach Anspruch 7, **dadurch gekennzeichnet**, daß das Kopplungselement (28) aus einer um die Schwenkachse (14) schwenkbaren Scheibe mit zwei in Axialrichtung entgegengesetzt vorstehenden Mitnehmerbolzen (24, 36) besteht, von denen einer (36) mit einem Ende der Feder (34) verbunden und der andere (24) in Anlage an die distale Schiene (1) bringbar ist.

9. Vorrichtung nach Anspruch 7 oder 8, **dadurch gekennzeichnet**, daß das Kopplungselement (28) an seinem Außenumfang sich radial nach innen erstreckende Aussparungen (29) aufweist, in die ein bezüglich der proximalen Schiene (2) verschiebbar gehalterter Arretierungsstift (31) einführbar ist.

## Claims

1. A device for the reduction of a deficit in extension or bending of a distal member of the body in relation to an adjacent proximal member of the body articulatingly connected with the distal body member, comprising splints (1 and 2) adapted to be connected with the distal and proximal body members , such splints being connected together by means of a rotary joint (3), a spring (34) being provided at the rotary joint (3) for acting between the distal and proximal splints (1 and 2), such spring producing in the free state of the spring (34), a biasing force in a predetermined direction of rotation, characterized in that a spring locking mechanism (28, 29 and 31) is provided, which is able to be pivoted between a non-locking state, wherein the biasing force of the spring (34) is able to be freely transmitted to the distal splint (1), and a locking state, wherein the biasing force of the spring (34) is decoupled from the distal splint (1) and is taken up by the spring force locking mechanism (28, 29 and 31) so that the distal splint (1) is able to be pivoted without any spring action over the free pivoting range.

2. The joint support as claimed in claim 1, characterized in that the spring (34) comprises a spiral spring surrounding the pivot axis pin (14), one end of the spiral spring being operatively connected with the one splint and the other end, in the free state of the spiral spring, being operatively connected with the other splint.

3. The joint support as claimed in claim 1 or in claim 2, characterized in that a biasing force modifying device (40 and 42) is provided which is in operative connected with one end of the spring (34), the relative position of such spring end being able to be modified in relation to the other spring end using such biasing force modifying device.

4. The joint support as claimed in claim 3, characterized in that the biasing force modifying device (40 and 42) comprises a gear wheel (40) which is able to be turned about the pivot axis pin (14) and is able to be locked in relation to one splint (2) by means of a gear wheel locking mechanism, one end of the spring (34) being secured to the gear wheel at a point near the pivot axis.

5. The joint support as claimed in claim 4, characterized in that the gear wheel locking mechanism comprises a ratchet pawl (45) for engagement with the teeth (41) of the gear wheel (40), such pawl preventing rotation of the gear wheel (40) in one direction of rotation and permitting it in the other direction of rotation.

6. The joint support as claimed in claim 4 or in claim 5 characterized in that a further actuating gear wheel (42) is in engagement with the gear wheel (40), such further actuating gear wheel having a smaller diameter, for constituting a transmission drive.

7. The joint support as claimed in any one of the preceding claims, characterized in that the spring force locking mechanism comprises a coupling element (28) arranged between the spring (34) and the distal splint (1) such coupling element (28) being on the one hand connected with one end of the spring (34) and on the other hand being able to be brought into engagement with the distal splint (1), the coupling element constantly urging, in its non-locked state, the distal splint (1) constantly in the desired direction of rotation by virtue of the biasing force, whereas in its locked state it is prevented from moving in this direction.

8. The joint support as claimed in claim 7, characterized in that the coupling element (28) comprises a disk able to be pivoted about the pivot axis (14) with two entrainment pins (24 and 36) projecting oppositely in the axial direction, of which one (36) is connected at one end of the spring (34) and the other (24) is able to be brought into engagement with the distal splint (1).

9. The joint support as claimed in claim 7 or claim 8, characterized in that on its outer periphery the coupling element (28) has radially inwardly extending recesses (29), into which a stp pin (31) is able to be introduced, such pin being held in a sliding manner in relation to the proximal splint (2).

## Revendications

1. Appareil pour la réduction des déficits en extension ou en flexion d'un membre corporel distal par rapport à un membre corporel proximal adjacent et relié par une articulation au membre corporel distal, comprenant des tringles (1, 2) susceptibles d'être fixées au membre corporel distal et au membre corporel proximal, lesdites tringles étant reliées l'une à l'autre par une articulation rotative (3), et il est prévu dans la région de l'articulation rotative (3) un ressort (34) qui agit entre la tringle distale et la tringle proximale (1, 2), de sorte que dans l'état libre du ressort (34) celui-ci exerce sur la tringle distale (1) une force de précontrainte dans une direction de rotation prédéterminée,
caractérisé en ce qu'il est prévu un mécanisme de blocage (28, 29, 31) vis-à-vis de la force du ressort, susceptible d'être passé depuis une situation de non-blocage, dans laquelle la force de précontrainte du ressort (34) est librement transmissible à la tringle distale (1), vers une situation de blocage dans laquelle la force de précontrainte du ressort (34) est découplée vis-à-vis de la tringle distale (1) et encaissée par le mécanisme de blocage de la force du ressort (28, 29, 31), de sorte que la tringle distale (1) est capable de pivoter sur une plage de pivotement libre sans action de la part du ressort.

2. Appareil selon la revendication 1, caractérisé en ce que le ressort (34) est un ressort spiralé qui entoure l'axe de pivotement (14), une extrémité du ressort spiralé étant activement reliée à l'une des tringles, et l'autre extrémité du ressort spiralé étant activement reliée, dans la situation libre, à l'autre tringle.

3. Appareil selon l'une ou l'autre des revendications 1 et 2, caractérisé en ce qu'il est prévu un dispositif de modification de forces de précontrainte (40, 42), en liaison d'action avec une extrémité du ressort (34), au moyen duquel la position relative de cette extrémité du ressort est modifiable par rapport à l'autre extrémité du ressort.

4. Appareil selon la revendication 3, caractérisé en ce que le dispositif de modification de forces de précontrainte (40, 42) comprend une roue dentée (40) capable de tourner autour de l'axe de pivotement (14) et susceptible d'être fixée par rapport à une tringle (2) au moyen d'un mécanisme de verrouillage (45), une extrémité du ressort (34) étant fixée sur ladite roue dentée dans une région proche de l'axe de rotation.

5. Appareil selon la revendication 4, caractérisé en ce que le mécanisme de verrouillage est constitué par un loquet de verrouillage (45) qui s'engage dans la denture (41) de la roue dentée (40), en bloquant la rotation de la roue dentée (40) dans une direction de rotation tout en permettant celle-ci dans l'autre direction de rotation.

6. Appareil selon l'une ou l'autre des revendications 4 et 5, caractérisé en ce qu'il est prévu une autre roue dentée d'actionnement (42), en engagement avec la roue dentée (40), pour constituer une transmission à démultiplication.

7. Appareil selon l'une des revendications précédentes, caractérisé en ce que le mécanisme de blocage de la force du ressort comprend un élément de couplage (28) agencé entre le ressort (34) et la tringle distale (1), ledit élément de couplage étant relié d'une part avec une extrémité du ressort (34), et pouvant d'autre part être amené en contact contre la tringle distale (1), ledit élément de couplage repoussant, dans son état de non blocage, la tringle distale (1) en permanence dans la direction de rotation souhaitée en raison de la force de précontraindre du ressort (34), tandis que dans sa situation bloquée il est empêché de se déplacer dans cette direction de rotation.

8. Appareil selon la revendication 7, caractérisé en ce que l'élément de couplage (28) est constitué par une plaque capable de pivoter autour de l'axe de pivotement (14), avec deux goujons d'entraînement (24, 36) dépassant en sens opposés en direction axiale, l'un desdits goujons (36) étant relié à une extrémité du ressort (34), tandis que l'autre (24) peut être amené en contact contre la tringle distale (1).

9. Appareil selon l'une ou l'autre des revendications 7 et 8, caractérisé en ce que l'élément d'accouplement (28) comporte à sa périphérie extérieure des évidements (29) qui s'étendent radialement vers l'intérieur et dans lesquels peut être introduite une tige d'arrêt (31) montée en translation par rapport à la tringle proximale (2).
